Europäisches Patentamt

European Patent Office

Office européen des brevets

Veröffentlichungsnummer: **0 286 958**
**A2**

## EUROPÄISCHE PATENTANMELDUNG

Anmeldenummer: 88105441.5

Int. Cl.⁴ **C12Q 1/68 , G01N 33/573**

Anmeldetag: 06.04.88

Priorität: 15.04.87 DE 3712786

Veröffentlichungstag der Anmeldung:
**19.10.88 Patentblatt 88/42**

Benannte Vertragsstaaten:
**BE CH DE FR GB IT LI NL SE**

Anmelder: **Merck Patent Gesellschaft mit beschränkter Haftung**
**Frankfurter Strasse 250**
**D-6100 Darmstadt(DE)**

Erfinder: **Reckmann, Bernd, Dr.**
**Freiligrathstrasse 7**
**D-6104 Seeheim(DE)**
Erfinder: **Rieke, Erwin, Dr.**
**Hermannstrasse 12**
**D-6104 Seeheim(DE)**

### Verfahren und Mittel zur Bestimmung von Nucleinsäuren.

Die Erfindung betrifft Verfahren und Mittel zur Bestimmung von Nucleinsäuren in einer Probe durch Überführung der zu bestimmenden Nucleinsäuresequenzen in Einzelstränge und Umsetzung der Einzelstränge mit einer komplementären Polynucleotidsonde. In der Polynucleotidsonde ist Cytidin durch 5-Aza-2'-desoxy-cytidin oder 5-Aza-cytidin ersetzt. Die Sonde wird nach Bindung mit gegebenenfalls markierter DNA-Methylase und Nachweise der Methylase über spezifische, gegebenenfalls markierte anti-Methylase-Antikörper oder über den Marker bestimmt.

EP 0 286 958 A2

### Verfahren und Mittel zur Bestimmung von Nucleinsäuren

Die Erfindung betrifft Verfahren und Mittel zur Bestimmung von Nucleinsäuren mit Hilfe von 5-Aza-2'-desoxycytidin (dazaC), DNA-Methylase und anti-DNA-Methylase Antikörpern.

Die Technik der DNA-Hybridisierung hat in den letzen Jahren zur Erforschung und Diagnose von genetischen und mikrobiologischen Erkrankungen breite Anwendung gefunden. Grundlage der DNA-Hybridisierung ist die Tatsache, daß zwei einzelsträngige komplementäre DNA-Moleküle unter Hybridisierungsbedingungen eine Doppelhelix bilden. In ähnlicher Weise entsteht aus einem einzelsträngigen DNA-Molekühl und einem RNA-Molekül ein DNA/RNA-Hybrid.

Zum Nachweis spezifischer Nucleinsäuresequenzen werden die in der Probe enthaltenen Nucleinsäuren durch Denaturierung in Einzelstränge zerlegt. Die entstandenen Einzelstränge werden anschließend auf eine inerte Oberfläche wie Nitrozellulose oder eine Nylonmembran fixiert. Die fixierten Einzelstränge werden mit einem komplementären Polynucleotid unterhalb der Dissoziationstemperatur in Kontakt gebracht, so daß eine Doppelstrangbildung aus den fixierten Einzelstrangsequenzen und der komplementären Polynucleotidsonde ermöglicht wird. Nach den Waschschritten wird der entstandene Doppelstrang aus Polynucleotidsonde und zu bestimmender Nucleinsäuresequenz nachgewiesen. Dazu wird die komplementäre Polynucleotidsonde in einer geeigneten Weise für die Wiederauffindung markiert. Üblicherweise erfolgt diese Markierung durch Einbau von Radioisotopen des Phosphors, Jods, Schwefels oder Wasserstoffs.

Ein großer Nachteil dieser Verfahren besteht in der Notwendigkeit des Umgangs mit radioaktiven Substanzen und den damit verbundenen Auflagen und Sicherheitsvorkehrungen. Es sind daher in der Vergangenheit vielfältige Versuche unternommen worden, die radioaktive Markierung von Nucleinsäuren durch nicht radioaktive Nachweissysteme zu ersetzen. So wurden Nucleotidtriphosphat-Analoge synthetisiert, die kovalent gebundenes Biotin an einem Pyrimidin-oder Purinring enthalten (EP 63879). Da diese Nucleotidderivate Substrate für RNA-und DNA-Polymerasen sind, können biotinmarkierte Polynucleotide enzymatisch hergestellt und zur Hybridiserung verwendet werden. Der Nachweis beruht auf der seit langem bekannten Avidin-Biotin-Wechselwirkung, wobei die gebildeten Doppelstränge mit einem Affinitätsnachweissystem (z.B. Avidin-Peroxidase) nachgewiesen werden. Dieses Verfahren hat jedoch den Nachteil, daß Biotin in sehr vielen biologischen Materialien vorhanden ist und sein Nachweis daher oft zu Störsignalen führt.

Weiterhin wurde in den letzten Jahren die Antigen-Antikörper-Wechselwirkung zum Nachweis von DNA-Hybridisierung eingesetzt. Zum Nachweis werden nach erfolgter Hybridisierung klassische immunologische Methoden, wie spezifische, ggf. markierte Antikörperenzymkonjugate eingesetzt. Das Antigen in dieser Antigen-Antikörper-Reaktion kann z.B. der gebildete Doppelstrang aus DNA oder DNA/RNA sein. So werden in der europäischen Patentanmeldung EP 135159 monoklonale Antikörper gegen doppelsträngige native DNA eingesetzt. In der europäischen Patentanmeldung EP 163220 wird eine Ribonucleinsäure als Sonde und spezifische anti-DNA/RNA-Hybrid-Antikörper eingesetzt. In beiden Fällen erfolgt der Nachweis der zu bestimmenden Nucleinsäure nach erfolgter Hybridisierung durch Bindung der spezifischen markierten Antikörper an die entstandenen DNA-Doppelhelices oder die DNA/RNA-oder RNA/RNA-Hybride.

Der schwerwiegende Nachteil des Einsatzes von spezifischen Antikörpern gegen native DNA liegt darin, daß bei der Hybridisierung der Polynucleotidsonde mit der nachzuweisenden DNA unter Hybridisierungsbedingungen auch Teile der nachzuweisenden DNA Doppelhelices ausbilden und damit zu einer unspezifischen Bindung der Antikörper führen. Ein weiterer Nachteil liegt in der Beschränkung auf Ribonucleinsäuren als Sonden, da die Verwendung von Ribonucleinsäuren besonders enge Bedingungen an die Hybridisierungsreaktion stellt, um eine Schädigung oder Zerstörung der Ribonucleinsäure durch einen zu hohen pH-Wert oder eingeschleppte Ribonuclease-Aktivität zu verhindern.

Das Antigen in der immunologischen Reaktion kann auch eine Polynucleotidsonde sein, die mit einem Hapten kovalent modifiziert ist. D.bei wird z.B. das Hapten über ein Brückenglied kovalent mit der Polynucleotidsonde verbunden (EP 173251). Der Nachteil dieser Methode liegt darin, daß das Hapten eine effiziente Hybridisierung zwischen der Polynucleotidsonde und der nachzuweisenden DNA einschränkt. Weniger voluminöse Haptene, wie sie in Form von modifizierten Nucleotidtriphosphaten auch enzymatisch in die DNA eingebaut werden können, bewirken keine so große Störung der Hybridisierungsreaktion. So ist z.B. die Verwendung von Bromuracil (EP 102228) und N-2-Acetylaminofluoren (EP 97664) bekannt. Der Nachweis der Hybridisierung erfolgt dann durch spezifische Antikörper gegen diese modifizierte Nucleotide enthaltende DNA. Allerdings sind die verwendeten modifizierten Nucleotide meist carcinogene Substanzen, deren Handhabung notwendigerweise mit Vorsichtsmaßnahmen und evtl. Gesundheitsrisiken verbunden ist.

Der Erfindung lag die Aufgabe zugrunde, ein neues Mittel und Verfahren zur Verfügung zu stellen, das die oben geschilderten Nachteile eliminiert.

Gelöst wird diese Aufgabe erfindungsgemäß durch ein Verfahren und Mittel zur Bestimmung von Nucleinsäuren mit Hilfe von DNA-Methylase, anti-DNA-Methylase Antikörpern und 5-Aza-2'-desoxycytidin oder 5-Aza-cytidin.

Gegenstand der Erfindung ist ein Verfahren zur Bestimmung von Nucleinsäuren in einer Probe durch Überführung der zu bestimmenden Nucleinsäuresequenzen in Einzelstränge und Umsetzung der Einzelstränge mit einer komplementären Polynucleotidsonde, das dadurch gekennzeichnet ist, daß in der Polynucleotidsonde Cytidin durch 5-Aza-2'-desoxycytidin oder 5-Aza-cytidin ersetzt ist und diese Sonde durch Bindung von gegebenenfalls markierter DNA-Methylase und Nachweise der Methylase über spezifische, gegebenenfalls markierte anti-Methylase-Antikörper oder über den Marker erfolgt.

Ein weiterer Gegenstand der Erfindung ist ein Mittel zur Bestimmung von Nucleinsäuren, das eine anstelle von Cytidin 5-Aza-2'-desoxicytidin oder 5-Aza-cytidin enthaltende Polynucleotidsonde, eine gegebenenfalls markierte Methylase und gegebenenfalls markierte anti-Methylase-Antikörper enthält. Die Polynucleotidsonde kann doppelstängig, einzelsträngig oder als Komplex mit der gegebenenfalls markierten Methylase vorliegen.

Überraschenderweise wurde gefunden, daß der Nachweis einer zu bestimmenden Nucleinsäurespecies dadurch gelingt, daß die hohe Bindungsaffinität der DNA-Methylase aus Säugetierzellen zum 5-Aza-cytidin ausgenutzt wird. Dazu wird in einer Polynucleotidsonde das d-Cytidin durch 5-Aza-2'-desoxycytidin ersetzt. Der Nachweis erfolgt durch Inkubation der entstandenen DNA-Hybride mit eukaryontischer DNA-Methyltransferase (= DNA-Methylase), wobei stabile dazaC-Methyltransferase-Komplexe gebildet werden. Diese Komplexe können dann durch spezifische Antikörper gegen DNA-Methyltransferase nach bekannten immunologischen Verfahren nachgewiesen werden.

Eukaryontische DNA-Methylase wird nach bekannten Verfahren aus z.B. menschlicher Placenta isoliert (Biochim. Biophys. Acta, 740, 323 (1983)). Das gereinigte Enzym ist ein 190-KD-Polypeptid, welches eine "Erhaltungs-" und eine "de novo" Methylase-Aktivität besitzt. Aufgrund dieser Aktivitäten bindet die DNA-Methylase sowohl an einzelsträngige als auch an doppelsträngige Nucleinsäuren. Die Bindung an mit dazaC modifizierter DNA ist aber wesentlich stabiler, so daß sie unter destabilisierenden Bedingungen, z.B. hohen Salzkonzentrationen, nicht dissoziert. Dadurch ist es überraschenderweise möglich, Bedingungen zu finden, die die unspezifische Bindung an Nucleinsäuren vollständig unterdrücken und nur die Bindung der Methylase an die dazaC-haltige DNA erlauben. Da die Komplexe aus Methylase und dazaC-haltiger DNA auch den rigiden Hybridisierungsbedingungen standhalten, eignen sich DNA-Methylase und dazaC hervorragend zum Nachweis von Hybridisierungsreaktionen.

Monoclonale Antikörper gegen eukaryontische DNA-Methylase werden nach bekannten Verfahren z.B. durch Immunisierung von Mäusen mit gereinigter Methylase und Konstruktion der Hybridomzellklone hergestellt (Eur. J. Cell. Biol. 34, 330 (1984)).

Die Herstellung einer dazaC-oder azaC-haltigen Polynucleotidprobe kann nach bekannten enzymatischen Methoden erfolgen, z.B. durch den Einbau von dazaCTP mittels Polymerase I (nick translation; Proc. Natl. Acad. Sci. USA 72, 1184 (1975)). Dazu muß allerdings das 5'-dazaC in das entsprechende Triphosphat überführt werden. Hierzu wird zu 5'-dazaC ein 1,5 molarer Überschuß $POCl_3$ bei 0 °C gegeben und 1 Std. unter Rühren bei 18 °C umgesetzt. Anschließend wird ein 2facher Überschuß Triethylammoniumpyrophosphat bei 0 °C zugegeben und 1 Std. bei 18 °C umgesetzt. Die Aufreinigung des gebildeten 5'-dazaCTP erfolgt über Cellulose-Dünnschichtchromatographie oder mittels HPLC.

Eine durch enzymatischen Einbau mit 5'-dazaCTP markierte DNA-Sonde kann in einzelsträngiger oder in doppelsträngiger Form in einen Hybridiserungsassay eingesetzt werden. 5-Aza-2'-desoxycytidin hydrolysiert unter bestimmten Bedingungen unter Ringöffnung zu Desoxyribofuranosyl-3-guanylharnstoff. Diese Hydrolyse erfolgt in doppelstängiger DNA langsamer als in einzelsträngiger DNA. Dieser Effekt kann dazu ausgenutzt werden, auf eine Abtrennung der nicht hybridisierten Polynucleotidsonde nach erfolgter Hybridiserung zu verzichten.

Der eigentliche Hybridisierungsschritt zwischen der erfindungsgemäß derivatisierten Nucleinsäure und der komplementären Nucleinsäure in der zu analysierenden Probe erfolgt nach bekannten Methoden. Bevorzugt ist die Hybridisierung auf einer festen Phase. Hierzu wird nach Aufspaltung der Nucleinsäure in Einzelstränge die nachzuweisende Nucleinsäure auf eine Festphase fixiert. Üblicherweise werden Nitrozellulose oder eine Nylonmembran eingesetzt. Anschließend wird die immobilisierte Nucleinsäure mit der dazaC enthaltenen Polynucleotidprobe unter Hybridisierungsbedingungen zusammengebracht.

Der Nachweis der Hybridisierung kann dann nach den üblichen Wasch-und Blockierschritten durch Zugabe und Inkubation mit DNA-Methylase erfolgen. Aufgrund der sehr hohen Bindungskonstante zwischen dazaC-haltiger DNA und der DNA-Methylase entsteht ein sehr stabiler Komplex aus nachzuweisender DNA, Polynucleotidsonde und der DNA-Methylase. Nach Entfernung der nicht gebundenen DNA-Methylase und der nicht hybridisierten Polynucleotidprobe kann die erfolgte Hybridisierung z.B. durch den Nachweis der gebundenen DNA-Methylase mit monoclonalen Maus-anti-DNA-Methylase-Antikörpern und einem mit β-Galactosidase konjugiertem zweiten anti-Maus-IgG nachgewiesen werden.

Die Verwendung von dazaC und DNA-Methylase bietet darüberhinaus eine Reihe weiterer Möglichkeiten, DNA-Hybridisierung einfach nachzuweisen. So besteht die Möglichkeit, gentechnologisch oder chemisch hergestellte DNA-Methylase-Enzym-Konjugate oder mit einem Marker gekoppelte DNA-Methylase zum Nachweis der gebildeten Hybride zu verwenden, wobei alle in bekannten Bindungstesten verwendeten Marker (Label) eingesetzt werden können. Dieses erspart die Inkubation mit dem anti-Methylase-Antikörper.

In einer anderen Ausführungsform wird die dazaC enthaltene Polynucleotidsonde vor der Hybridisierung mit DNA-Methylase inkubiert und die gebildeten DNA-Methylase-Polynucleotidsonden-Komplexe werden zur Hybridisierung eingesetzt. Der Nachweis der Hybridisierung mit der nachzuweisenden Nucleinsäure erfolgt auch hier nach Durchführung der Hybridisierung und der üblichen Waschschritte durch Bindung von anti-DNA-Methylase-Antikörpern, an die Methylase nach bekannten Verfahren. Dieser Schritt kann entfallen, wenn direkt ein Komplex aus Polynucleotidsonde und mit einem Marker oder mit einem Enzym gekoppelter DNA-Methylase verwendet wird.

Des weiteren besteht die Möglichkeit, die dazaC-haltige Polynucleotidsonde nach Hybridisierung mit der nachzuweisenden DNA mit anti-dazaC-Antikörpern direkt zu inkubieren. Der Nachweis der Hybridisierung erfolgt dann nach bekannten immunologischen Verfahren mittels eines zweiten mit einem Marker versehenen Antikörpers oder direkt, falls der anti-dazaC-Antikörper mit einem Marker oder Enzym gekoppelt ist.

Da die DNA-Methylase auch an natürliche DNA bindet, kann leicht ein kovalenter Komplex aus DNA-Methylase oder mit einem Marker oder Enzym gekoppelter Methylase und natürlicher einzelsträngiger bzw. doppelsträngiger DNA durch Bestrahlen im nahen UV-Bereich oder durch andere bekannte chemische Vernetzungsreaktionen zwischen Protein und Nucleinsäuren gebildet werden. Dieser Komplex kann dann direkt zur Hybridisierung eingesetzt und in der üblichen Weise nachgewiesen werden.

Die Modifikation der Nucleinsäure durch den Einbau des dazaCTP beeinflußt die Hybridisierungsreaktion nicht, darüberhinaus werden die in der Hybridisierungsreaktion gebildeten DNA DNA-Hybride durch die anschließende Bindung der DNA-Methylase verstärkt. Eine weitere Sensitivitätssteigerung wird dadurch möglich, daß auf der DNA-Methylase mehrere Epitope die spezifischen Antikörper binden können, so daß eine zusätzliche Amplifikation entsteht.

Mit dem erfindungsgemäßen Verfahren lassen sich auf einfache Weise Analysenergebnisse erzielen, die den bekannten Verfahren zur Nucleinsäurebestimmung mindestens ebenbürtig sind. Aufgrund der sehr hohen Bindungskonstante von DNA-Methylase an dazaC-haltige DNA sind Nachweise auch in sehr geringen Konzentrationsbereichen möglich, ohne daß es zu einer störenden unspezifischen Bindung von DNA-Methylase an nicht dazaC-haltige DNA komit.

Beispiel 1

Herstellung einer dazaC-haltigen Polynucleotidsonde durch "nick translation"

1 μg Plasmid-DNA (pBCam 3225) wird in einem Volumen von 50 μl unter Standardbedingungen (je 50 μM dATP, dGTP, dTTP, 1 μM dCTP, 5 mM MnCl$_2$, 50 μg/ml BSA, 50 mM Tris-HCl, pH 7.5) mit 500 μM dazaCTP und 2 pg DNAse I und 500 U DNA Polymerase I bei 15 °C inkubiert. Nach einer Stunde wird die Reaktion durch Zugabe von 100 μl 12,5 mM EDTA, 0,5 % Natriumdodecylsulfat (SDS) abgebrochen. Um die mit azaC modifizierte DNA von den nicht umgesetzten Nucleotidtriphosphaten abzutrennen, wird der Ansatz über eine Sephadex G-50 Säule chromatographiert. Die DNA läuft im Ausschlußvolumen und wird in 400 μl 10 mM Tris-Puffer, pH 7,6, eluiert.

Beispiel 2

Herstellung einer einzelsträngigen dazaC-haltigen Polynucleotidsonde

5 pmol einzelsträngiger M13camp5-DNA werden mit 5 pmol eines synthetisierten Oligonucleotids (60 N), welches komplementär zur in M13camp5 klonierten Campylobacter-DNA ist, hybridisiert. Der entstandene Primer-Template-Komplex wird mit 100 U DNA-Polymerase I (Klenow-Enzym) und je 200 μM dGTP, dATP, dTTP, 5 μM dCTP, 750 μM dazaCTP in 50 μl 5 mM MnCl$_2$, 50 mM Tris-HCl, pH 7,5 und 50 μg/ml BSA bei 15 °C inkubiert. Nach 45 Min. wird die Reaktion gestoppt und der Ansatz auf einem 8 % denaturierenden Polyacrylamid-Gel aufgetrennt. Einzelständige dazaC-haltige DNA, die größer als 150 N ist, wird eluiert und zur Hybridisierung eingesetzt.

### Beispiel 3

a) Hybridisierung mit dazaC-haltiger Polynucleotidsonde

Auf eine Nylonmembran werden mittels einer dot-blot Apparatur verschiedene Mengen denaturierte Campylobacter-DNA aufgetragen (10 ng · 1 pg). Nach Prehybridisierung des Filters für 1 Std. bei 60 ° in 6×SSC (Natriumchlorid/Natriumcitrat), 5× Denhardt (Rinderserumalbumin/Polyvinylpyrrolidon/Ficoll),0,5 % SDS und 20 $\mu$g/ml denaturierte Heringssperma-DNA erfolgt durch Zugabe der aus Beispiel 1 nick translatierten dazaC-haltigen DNA-Sonde in einer Konzentration von 300 ng/ml die eigentliche Hybridisierung für 3 Std. bei 60 °C. Anschließend wird das Filter nach bekannten Verfahren mit abnehmenden SSC-Konzentrationen bis zur gewünschten Stringenz gewaschen.

b) Bindung von DNA-Methylase an DNA-dazaC-DNA-Hybride

Zum Nachweis des in der Hybridisierungsreaktion gebildeten Hybrids aus nachzuweisender DNA und der dazaC-haltigen DNA-Sonde werden die Hybride mit DNA-Methylase inkubiert. Dazu wird die Nylonmembran unter Schütteln mit 1 $\mu$g/ml DNA-Methylase in 20 mM Tris-Puffer, pH 7,5, 5 mM EDTA, 0,5 mM Dithioerythrol, 2 % Glycerin, 1 % BSA, 10 $\mu$M S-Adenosyl-Methionin bei 37 °C 15 Min. inkubiert. Nicht gebundene DNA-Methylase wird durch je 5minütiges Waschen des Filters mit 20 mM Tris-Puffer, pH 7,5, 5 mM EDTA, 0,4 M NaCl und anschließend mit 20 mM Tris-Puffer, pH 7,5, 5 mM EDTA,0,1 M NaCl bei Raumtemperatur entfernt.

c) Nachweis der gebundenen Methylase durch monoclonale Maus-anti-DNA-Methylase-Antikörper und sekundären $\beta$-Galactosidase konjugiertem anti-Maus-IgG

Nach Waschen der Membran aus b) mit 1× PBS (Natriumphosphat), pH 7,5 und 0,1 % Tween 20 wird mit dem Maus-anti-DNA-Methylase-Antikörper,(1 $\mu$g/ml) 30 Min. bei Raumtemperatur in 1× PBS, 0,1 % BSA inkubiert. Anschließend wird der $\beta$-Galactosidase-anti-Maus-IgG-Antikörper zugesetzt und ebenfalls 30 Min. bei Raumtemperatur inkubiert. Die nicht gebundenen Antikörper werden durch 5maliges Wischen mit 1× PBS entfernt. Anschließend wird mit der Enzymsubstratlösung (0,5 mg/ml 5-Brom-4-Chlor-3-Indolyl-Galactosid, 1 mM mgCl$_2$, 3 mM Kaliumferricyanid, 3 mM Ferrocyanid in PBS, pH 7,5) mindestens 1 Std. bei Raumtemperatur inkubiert. Auf diese Weise lassen sich problemlos 50 pg Campylobacter-DNA nachweisen.

### Beispiel 4

Inkubation einer einzelsträngigen dazaC-haltigen DNA-Sonde mit DNA-Methylase

Die in Beispiel 2 hergestellte einzelsträngige dazaC-haltige DNA-Sonde wird mit DNA-Methylase (10 $\mu$g/ml), wie in Beispiel 3b beschrieben, inkubiert. Zur teilweisen Abtrennung ungebundener Methylase wird der Ansatz über eine Sephadex G-200 Säule chromatographiert. Der erhaltene DNA-Sonde-Methylase-Komplex ist äußerst stabil und kann zum Nachweis von Campylobacter-DNA, wie in Beispiel 3a be schrieben, eingesetzt werden. Die Hybridisierungstemperatur liegt dabei 35 °C unter der erwarteten Dissoziationstemperatur des nicht mit DNA-Methylase gelabelten Sonde-DNA-Hybrids. Der Nachweis der erfolgten Hybridisierung erfolgt wie in Beispiel 3c beschrieben.

### Beispiel 5

Nachweis der Hydridisierung mit einem DNA-Methylase-Peroxidase-Konjugat

Zum Nachweis des in Beispiel 3 gebildeten Hybrids aus nachzuweisender DNA und der dazaC-haltigen DNA-Sonde wird mit einem DNA-Methylase-Peroxidase-Konjugat inkubiert. Die Inkubation erfolgt analog der Inkubation mit DNA-Methylase, wie in Beispiel 3b beschrieben.

Nach Bindung und Abtrennung des nicht gebundenen DNA-Methylase-Enzym-Konjugats erfolgt der direkte Nachweis der Hybridisierung durch Zugabe der Peroxidasesubstratlösung aus 0,025 % Wasserstoffperoxid und 400 $\mu$g/ml Aminoethylcarbazol in 100 mM Natriumacetatpuffer, pH 4,5, und Inkubation bei Raumtemperatur, bis eine deutliche Farbreaktion auftritt.

Die Herstellung des Konjugates aus DNA-Methylase und Meerrettich-Peroxidase erfolgt nach bekannten Verfahren mit dem heterobifunktionellen Kopplungsreagenz N-Succinimidyl-3-(2-pyridyldithio)-propionat.

## Ansprüche

1. Verfahren zur Bestimmung von Nucleinsäuren in einer Probe durch Überführung der zu bestimmenden Nucleinsäuresequenzen in Einzelstränge und Umsetzung der Einzelstränge mit einer komplementären Polynucleotidsonde, dadurch gekennzeichnet, daß in der Polynucleotidsonde Cytidin durch 5-Aza-2'-desoxy-cytidin oder 5-Aza-cytidin ersetzt ist und diese Sonde durch Bindung von gegebenenfalls markierter DNA-Methylase und Nachweise der Methylase über spezifische, gegebenenfalls markierte anti-Methylase-Antikörper oder über den Marker erfolgt.

2. Verfahren nach Anspruch 1, dadurch gekennzeichnet, daß der DNA-Methylase-DNA-Sonden-Komplex durch Maus-anti-DNA-Methylase-Antikörper und einen Maus-IgG-Antikörper oder durch ein anti-Maus-IgG Antikörper-Enzymkonjugat nachgewiesen wird.

3. Verfahren nach Anspruch 1, dadurch gekennzeichnet, daß eine 5-Aza-2'-desoxycytidin-oder eine 5-Aza-cytidinhaltige Polynucleotidsonde durch spezifische gegebenenfalls markierte Antikörper oder Antikörper-Enzymkonjugate gegen 5-Aza-2'-desoxycytidin-oder 5-Aza-cytidinhaltige Nucleinsäure nachgewiesen wird.

4. Verfahren nach den Ansprüchen 1 bis 3, dadurch gekennzeichnet, daß die nachzuweisende Nucleinsäure direkt oder über eine immobilisierte, komplementäre Nucleinsäure an eine feste Phase gebunden wird.

5. Mittel zur Bestimmung von Nucleinsäuren, enthaltend eine anstelle von Cytidin 5-Aza-2'-desoxycytidin oder 5-Aza-cytidin enthaltende Polynucleotidsonde, eine gegebenenfalls markierte Methylase und gegebenenfalls markierte anti-Methylase-Antikörper.

6. Mittel nach Anspruch 5, dadurch gekennzeichnet, daß die Polynucleotidsonde doppelsträngig, einzelsträngig oder als Komplex mit der gegebenenfalls markierten Methylase vorliegt.